Europäisches Patentamt

European Patent Office

Office européen des brevets

(11) Publication number: **0 240 191**
**A2**

# EUROPEAN PATENT APPLICATION

(21) Application number: 87302138.0

(51) Int. Cl.⁴: **C12N 15/00 , C12P 19/34**

(22) Date of filing: 12.03.87

(30) Priority: 13.03.86 JP 55544/86
04.04.86 JP 77853/86

(43) Date of publication of application:
07.10.87 Bulletin 87/41

(84) Designated Contracting States:
DE FR GB

(71) Applicant: SEIKO INSTRUMENTS INC.
31-1, Kameido 6-chome Koto-ku
Tokyo 136(JP)

(72) Inventor: Masafumi, Murata
c/o Seiko Instruments Inc. 31-1, Kameido
6-chome
Koto-ku Tokyo(JP)
Inventor: Munechika, Sakaba
c/o Seiko Instruments Inc. 31-1, Kameido
6-chome
Koto-ku Tokyo(JP)

(74) Representative: Caro, William Egerton et al
J. MILLER & CO. Lincoln House 296-302 High
Holborn
London WC1V 7JH(GB)

(54) Method of isolating DNA contained in a virus or cell.

(57) A method of isolating DNA contained in a virus or a cell comprises the steps of; isolating the virus or cell having satellite DNA ; reacting a protein decomposer with said virus or cell for destroying or denaturing protein covering DNA; adding at least twice by volume of alcohol to the reaction mixture for precipitating DNA; feeding an alcohol containing solution obtained in the previous step into a separation column for retaining the precipitated DNA; passing an aqueous alcohol solution having a high alcohol concentration through the column for removing impurity; and passing an aqueous solution having a low alcohol concentration or an aqueous solution free from alcohol through said column for eluating said DNA.

## METHOD OF ISOLATING DNA CONTAINED IN A VIRUS OR CELL

This invention relates to methods of isolating DNA contained in viruses or cells, eg to methods of obtaining virus DNA or small circular double stranded satellite DNA in cells.

Conventionally, isolation of DNA contained in a virus particle has extensively been conducted to study the virus itself or to obtain a virus vector for a recombinant DNA. The conventional methods comprised repetition of a liquid-liquid partition and centrifugation. Examples of such methods include a method of isolating a vaccinia virus DNA as described by M Mackett, G L Smith and B Moss in "DNA Cloning", Vol 2, p. 204, published by IRL Press and a method of isolating an M 13 bacteriophage DNA as described by Haruo Omori in "Kakusan Tanpakushitsu Kozo Kaiseki Giho (Structural Analysis Techniques for Nucleic Acids and Proteins)", p. 33, published by Science Co Limited.

On the other hand, isolation of small circular double-stranded satellite DNA contained within a prokaryotic cell particle has been essential to genetic engineering and has extensively been conducted to obtain a vector for a recombinant DNA or to obtain a sample for use in study of small circular double-stranded satellite DNA itself, and a number of methods have been proposed therefor. All the conventional methods required the use of a centrifuge. Examples of such methods includes a method as described by T Maniatis et al. in "Molecular Cloning" published in 1982 by Cold Spring Harbor Lab, which is one of the methods which uses a centrifuge most often. This method is described particularly as a method of isolating a plasmid from **Escherichia coli.**

In all the above-mentioned conventional methods, the separation into precipitate and supernatant was conducted with a centrifuge without use of any filter, and the supernatant was collected by decantation into a new vessel, while the precipitate was collected by removing the supernatant. Therefore, the conventional methods required much work, prevented researchers from doing intellectual thinking for long periods of time and frequently required continuation of work until midnight. The conventional methods are not only complicated procedures and need a large amount of time but also may cause DNA to be cleaved, because they use a centrifuge repeatedly and discard and transfer the supernatant and aqueous phase by means of decantation or pipetting.

The present invention seeks to provide a method of isolating DNA contained in a virus or cell without centrifugation and extraction procedures, in a relatively short period, and without cell cleavage.

According to the present invention there is provided a method of isolating DNA contained in a virus or a cell comprising the steps of: isolating the virus or cell having satellite DNA; reacting a protein decomposer with said virus or cell for destroying or denaturing protein covering DNA; adding at least twice by volume of alcohol to the reaction mixture to precipitate the DNA; feeding an alcohol containing solution obtained in the previous step into a separation column for retaining the precipitated DNA; passing an aqueous alcohol solution having a high alcohol concentration through said column to remove impurity; and passing an aqueous solution having a low alcohol concentration or an aqueous solution free from alcohol through said column for eluating said DNA.

In one embodiment the method includes the steps of: adding a virus particle-aggregating agent, eg polyethylene glycol to a double suspension comprising a virus and a hose cell therefor, subjecting the mixture containing an aggregated virus and host cell obtained in the previous step to filtration with a filter to filter off said aggregated virus and host cell, and eluating said virus with water or a buffer from said filter.

In another embodiment isolation of a cell is conducted by filtration.

The invention will now be described, merely by way of example, with reference to the following Examples.

### EXAMPLE 1

A method according to the present invention for isolating DNA contained in a virus particle includes the addition of a virus particle-aggregating agent, eg, polyethylene glycol, to a starting material, ie, a double suspension comprising a virus particle and a host cell, causing the virus particle to aggregate to form a large particle. The aggregated virus particle and host cell can easily be filtered off by means of a filter. When water or a buffer is passed through the filter which holds the aggregated virus particle and host cell, the water or buffer eluates the virus-aggregating agent first and then the virus particle which is in such a state that the aggregate has disintegrated, which enables a virus particle solution free from the host cell to be obtained.

The addition of a protein decomposer or protein denaturing agent such as proteinase K or SDS to the virus particle solution causes the virus to be separated into a decomposition product or denaturation product of a coat protein and DNA. The subsequent addition of ethanol thereto brings about precipitation of DNA. When the solution containing the precipitated DNA is passed through a column, the precipitated DNA is trapped in the column. The DNA is washed with an aqueous ethanol solution having a high ethanol concentration, eluted by means of an aqueous solution having a low ethanol concentration or an aqueous solution free from ethanol and withdrawn from the column to collect a DNA fraction.

A preferred embodiment of the present Example will now be described using **Escherichia coli.**

**Escherichia coli** infected with M13 phage and **Escherichia coli** infected with lambda phage which had been incubated according to the customary method were used in an amount of 2 ml as starting material. At the outset, in order to aggregate the phage, an aqueous solution containing 200 microlitres of 20% polyethylene glycol C6000 and 2.5 M NaCl was added. The resulting solutions each were passed through a filter unit equipped with a membrane filter made of polyvinylidene fluoride and having a pore diameter of 0.22 microns and a diameter of 25mm to filter off the aggregated phage and **Escherichia coli.** 400 microlitres of a buffer containing 10mM Tris-HCl (pH 7.8) and 1mM EDTA was passed through each filter, causing the phage to be transferred to the buffer. 10 microlitres of SDS and 20 micrograms of proteinase K were added to the permeated solutions, respectively, and the solutions were then allowed to stand at 55°C for 15 min. Thereafter, 1 ml of ethanol was added to each solution. The solutions were then passed through miniature columns each packed with CF11 cellulose, respectively. In this step, DNA was held in the miniature column. 100 microlitres of a buffer containing 10mM Tris-HCl (pH 7.8) and 1mM EDTA was passed through each miniature column to eluate DNA. In order to compare DNAs thus obtained with DNA which has been obtained by a customary method, each sample was stained with ethidium bromide. As a result, it was found that no difference existed between the amounts of DNAs collected above and that collected by the customary method;. Further, the stained DNAs were applied to agar gel electrophoresis to examine the patterns. As a result, it was found that the band thus obtained was single, ie, the DNAs were free from other nucleic acids as impurities.

As is apparent from this Example DNA having a satisfactory purity can surely be obtained in a short period of time.

### Example 2

A water-soluble impurity-containing suspension of a prokaryotic cell particle which is a starting material of a method according to the present invention for double-stranded satellite DNA may be of various kinds and includes cultures of autotrophic prokaryotes, ie, blue-green algae, photosynthetic bacteria and chemosynthetic bacteria. They contain only inorganic salts besides cell debris. Mitochondrion, chloroplast, etc of a eukaryote are regarded as a prokaryotic cell particle judging from their constitutions. The suspensions thereof are those which have been collected from a destroyed eukaryotic cell. Therefore, they are already suspended in a buffer composed substantially of an inorganic salt solution. Heterotrophic prokaryotes are suspended in a medium containing nutrients such as sugars, amino acids and vitamins. The minimum diameters of prokaryotic cell particles contained in various suspensions as mentioned above are known. When a water-soluble impurity-containing suspension of the above mentioned prokaryotic cell particle is filtered with a surface trap filter having pores smaller than that of the minimum diameter, the prokaryotic cell particle is left on the filter while the solution in which the prokaryotic cell particle was suspended permeates through the filter and is discharged. The subsequent immersion of the filter, on which the prokaryotic cell particle has been held, in a lysing solution brings bout destruction of the prokaryotic cell particle, which leads to the elution of the water-soluble components into the lysing solution. When the prokaryotic cell particle has a cell wall, a cell wall lytic enzyme solution is first used to remove the cell wall, and an alkaline solution is later added to the cell wall lytic enzyme solution containing prokaryotic cell particle which is in a spheroplast form to inactivate the cell wall lytic enzyme by hydrolysis and, at the same time, to cause lysis and to hydrolyze protein components which have been eluated accompanying the lysis. On the other hand, when the prokaryotic cell particle has no cell wall, only an alkaline solution is used as the lysing solution. In the step of adding a solution having a high salt concentration, the water-soluble components capable of being salted out are precipitated, and the alkali is neutralized. In this step, a high-molecular chromosomal DNA turns into a supercoiled state and can be precipitated together with RNA. The precipitate which has been formed by addition of the above-mentioned solution having a high salt concentration is removed by filtration with a filter from the permeated solution. When ethanol is added in the final step, most of the salts are dissolved in ethanol while the small circular double-stranded DNA precipitates due to the presence of ethanol. Therefore, the small circular double-stranded satellite DNA can be collected with a trace amount of impurities in a solid state by removing the supernatant.

The supernatant is removed in the same way as described in Example 1. A preferred embodiment of the present Example will now be described using **Escherichia coli** containing plasmid pBR322.

1) 5 ml of a culture of **Escherichia coli** containing plasmid pBR322 which had been incubated according to a customary method was taken out with a syringe. A membrane filter unit made of polyvinylidene fluoride and having a pore diameter of 0.22 microns and a diameter of 25mm was connected to the syringe. The piston of the syringe was pushed in to leave the cell debris in the culture on the membrane filter and to remove the medium.

2) The syringe was disconnected, charged with 5 ml of a buffer containing 50mM glucose, 10mM EDTA, and 25mM Tris-HCl (pH 8.0) and again connected to the membrane filter unit on which the cell debris had been held. The piston of the syringe was pushed in to wash the cell debris and the inside of the membrane filter on which the cell debris had been held.

3) The syringe which had been connected to the membrane filter holding the cell debris thereon was charged with 0.2 microlitres of a cell wall lytic enzyme solution containing 2 mg/ml of lysozyme, 50mM glucose, 10mM EDTA, and 25mM Tris-HCl (pH 8.0). The cell wall lytic enzyme solution was pushed out and kept at room temperature for 20 min. The syringe was replaced with a syringe charged with 0.4 ml of a solution of 0.2 N NaOH and 1% SDS, and half of the solution was pushed out on the membrane filter unit. The discharging pore of the membrane filter unit was clogged with a lab film. The membrane filter unit was allowed to stand in iced water for 5 min.

4) The solutions contained in the above-mentioned syringe and membrane filter unit were discharged in a 2-ml plastic test tube. The addition of 0.3 ml of a solution containing 3 M sodium acetate and acetic acid (pH 4.8) caused formation of an aqueous solution containing a white precipitate.

5) The aqueous solution containing the white precipitate was treated with a fresh membrane filter unit and syringe which are of the same type as used above, and 0.5 ml of the resulting filtrate was placed in a new 2-ml plastic test tube.

6) Twice the volume of ethanol was added to the filtrate, and the mixture was allowed to stand in a freezer kept at -70°C below zero for 30 min. The mixture was passed through a cellulose miniature column, and 100 microlitres of a TE buffer was passed therethrough for elution. The resulting eluate was stained with ethidium bromide. It developed a colour which appeared to suggest the presence of DNA. Part of the eluate was digested with a restriction enzyme, Taq I, and then stained with ethidium bromide.

It was electrophoresed simultaneously with a solution obtained by subjecting pBR 322 to the same treatment as mentioned above and a solution obtained by staining the eluate with ethidium bromide without digesting with the restriction enzyme to examine patterns. As a result, it was found that the sample obtained by digesting the above-mentioned aqueous solution containing precipitate with Taq I exhibited seven bands at the same sites as the sample obtained by digesting pBR with Taq I. On the other hand, the sample which had not been subjected to any digestion with the restriction enzyme exhibited only one band. It was confirmed from the above facts that the precipitate obtained in the present example was pBR 322 contained in the cell debris.

As is apparent from this Example small circular double-stranded satellite DNA can be obtained without complicated procedures.

In this example, step 4 and step 5 can be omitted if extra pure DNA is not required.

## Claims

1. A method of isolating DNA contained in a virus or a cell characterised by comprising the steps of:
isolating the virus or cell having satellite DNA; reacting a protein decomposer with said virus or cell for destroying or denaturing protein covering DNA; adding at least twice by volume of alcohol to the reaction mixture to precipitate the DNA; feeding an alcohol containing solution obtained in the previous step into a separation column for retaining the precipitated DNA; passing an aqueous alcohol solution having a high alcohol concentration through said column to remove impurity; and passing an aqueous solution having a low alcohol concentration or an aqueous solution free from alcohol through said column for eluting said DNA.

2. A method as claimed in claim 1 characterised by comprising the steps of adding a virus particle aggregating agent to a double suspension comprising a virus and a host cell therefor, subjecting the mixture containing an aggregated virus and host cell obtained in the previous step to filtration with a filter to filter off said aggregated virus and host cell, and eluating said virus with water or a buffer from said filter.

3. A method as claimed in claim 2 characterised in that the virus particle-aggregating agent is polyethylene glycol.

4. A method as claimed in claim 1 characterised in that isolation of a cell is conducted by filtration.